# EUROPEAN PATENT APPLICATION

(11) **EP 4 173 629 A1**
(43) Date of publication of application: **03.05.2023**
(21) Application number: 21832276.6
(22) Date of filing: 30.06.2021
(51) Int. Cl.: A61K 35/28, A61K 9/19, A61K 35/50, A61K 35/51, A61K 38/19, A61K 38/20, A61P 7/00, A61P 9/00, A61P 21/00, A61P 25/00, A61P 25/02, A61P 25/08, A61P 25/14, A61P 25/16, A61P 25/18, A61P 25/20, A61P 25/28, A61P 39/02, A61P 43/00

(54) **CRANIAL NERVE DISORDER THERAPEUTIC AGENT INCLUDING CULTURE SUPERNATANT OF TISSUE CELLS DERIVED FROM FETAL APPENDAGE**

(30) Priority: 30.06.2020 JP 2020113456; 07.09.2020 JP 2020149878
(71) Applicant: National University Corporation Kochi University, Kochi-shi, Kochi 780-8520 (JP)
(72) Inventor: SAGARA, Yusuke, Kochi-shi, Kochi 780-8520 (JP)
(74) Representative: advotec.
(86) International application number: PCT/JP2021/024837
(87) International publication number: WO 2022/004816

(57) **Abstract**

Provided is a cranial nerve disorder therapeutic agent that includes, as an active ingredient, a culture supernatant of tissue cells derived from a fetal appendage.

## Description

### FIELD

The present invention relates to a therapeutic agent for brain disorder comprising a culture supernatant of tissue cells derived from a fetal appendage.

### BACKGROUND

Neonatal brain disorders during the perinatal period occur at a frequency of 1 or 2 per 1000 child births, causing lifelong cerebral palsy and creating a major burden not only on the individual but also on the family that raises them.

The perinatal brain injury that is causative of cerebral palsy includes hypoxic-ischemic encephalopathy, cerebral hemorrhage and periventricular leukomalacia, the major pathologies being intracellular Ca increase, active oxygen increase caused by mitochondrial function insufficiency, and inflammation of hypercytokinemia accompanying macrophage activation. Cerebral hemorrhage is a common complication of premature infancy, and its symptoms are the most severe with poor life prognosis.

Hypothermia treatment is considered to be an effective method, but has an efficacy rate of only about one in 8 to 9 infants. No method of treatment other than hypothermia has yet been established.

Clinical research is progressing in the area of direct administration of autologous bone marrow-derived mesenchymal cells into cerebral palsy patients, with some efficacy being reported (NPL 1). Administration of umbilical cord-derived cells has also been reported, but no significant improvements have been observed in certain motor functions (NPL 2). Significant amelioration in motor function impairment has also been reported by administration of umbilical cord blood-derived cells to neonatal cerebral hemorrhage model mice (PTL 1). Transplantation of umbilical cord- and placenta-derived cells to optic nerve injury model mice has been reported to promote regrowth of the optic nerve (PTL 2).

### [CITATION LIST]

### [NON PATENT LITERATURE]

[NPL 1] Cytotherapy 2013 Dec; 15(12):1549-62
[NPL 2] Cytotherapy 2015 17(2): 224-231

### [PATENT LITERATURE]

[PTL 1] International Patent Publication No. WO2017/204231
[PTL 2] Japanese Patent Public Inspection No. 2007-521793

### SUMMARY

### [TECHNICAL PROBLEM]

In cerebral hemorrhagic or ischemic cranial nerve disorder, it is necessary not only to cure the hemorrhage and ischemia cause but also to protect and promote regeneration of the neurons that are damaged as a result. It has been considered to administer umbilical cord-derived cells for protection and regeneration of such neurons, but due to the different characteristics of cells obtained by methods of harvesting from umbilical cords, it has been unclear what effects are to be obtained by different types of cells derived from umbilical cords or from different donors. Intrasubarachnoid administration can also be conducted by lumbar puncture for cranial nerve disorder, but this method of administration is risky and therefore therapeutic agents that exhibit effects by easier routes of administration are desired.

### [SOLUTION TO PROBLEM]

The present inventors have conducted much diligent research with the aim of solving the problems described above, and have completed this invention upon finding that a culture supernatant of tissue cells derived from fetal appendage promotes proliferation and survival of cranial neural progenitor cells, homing to brain disorder sites and differentiation and maturation of cranial neural progenitor cells, and thereby regenerates cranial nerves and is effective for cranial nerve disorder.

Specifically, the invention relates to the following:
(1) A cranial nerve disorder therapeutic agent comprising a culture supernatant of tissue cells derived from fetal appendage as an active ingredient.
(2) The cranial nerve disorder therapeutic agent according to (1), wherein the tissue cells derived from fetal appendage are umbilical cord cells, placental cells, egg membrane cells, chorionic cells, amniotic cells or a combination thereof.
(3) The cranial nerve disorder therapeutic agent according to (1) or (2), wherein the tissue cells derived from fetal appendage are Walton colloid-derived mesenchymal cells, amnionic mesenchymal cells or chorionic mesenchymal cells, or a combination thereof.
(4) The cranial nerve disorder therapeutic agent according to any one of (1) to (3), wherein the culture supernatant comprises cytokines and chemokines.
(5) The cranial nerve disorder therapeutic agent according to any one of (1) to (4), comprising IL-6, CXCL1, CXCL7, CXCL8 and CCL2.
(6) The cranial nerve disorder therapeutic agent according to any one of (1) to (5), wherein the culture supernatant is obtained by culturing tissue cells derived from fetal appendage for 1 to 3 days.
(7) The cranial nerve disorder therapeutic agent according to any one of (1) to (6), wherein the density of tissue cells derived from fetal appendage is 5 × 10⁴ to 5 × 10⁶/mL at the start of culturing.
(8) The cranial nerve disorder therapeutic agent according to any one of (1) to (7), wherein the cranial nerve disorder is non-hereditary cranial nerve disorder.
(9) The cranial nerve disorder therapeutic agent according to any one of (1) to (8), wherein the cranial nerve disorder is dyskinesia (levodopa-induced dyskinesia, chronic or tardive dyskinesia, or orofacial dyskinesia), restless leg syndrome (drug-induced or idiopathic), drug-induced dystonia, chorea (Huntington's disease, toxin-induced chorea, Sydenham chorea, chorea of pregnancy, Wilson's disease, drug-induced chorea or metabolic or endocrine chorea), facial spasm (such as mobile, phonetic, simple, complex or Tourette's syndrome), dystonia (such as acute, systemic, localized, segmental, sexual, neutral, psychogenic or acute dystonic reaction), Sodemytopic Parkinson's disease, stereotypic movement disorder (such as autism or heredity or childhood-related movement disorder), obsessive-compulsive disorder, narcolepsy (such as cataplexy), transmissible spongiform encephalopathy (such as Creutzfeldt-Jakob disease or kuru), dementia (such as Alzheimer's, Lewy body dementia, vascular dementia, Pick's disease or alcoholic dementia), neuroacanthocytosis, seizure or convulsion, athetosis (such as Huntington's disease, respiratory arrest, neonatal jaundice- or stroke-related), or cerebral palsy.
(10) A method for producing a cranial nerve disorder therapeutic agent according to any one of (1) to (9), the method including (a) a step of culturing tissue cells derived from fetal appendage, and (b) a step of recovering the culture supernatant of the tissue cells derived from fetal appendage.
(11) A pharmaceutical composition comprising a cranial nerve disorder therapeutic agent according to any one of (1) to (9).
(12) The pharmaceutical composition according to (11), which is a lyophilized preparation.
(13) A cranial nerve regeneration promoter comprising a culture supernatant of tissue cells derived from fetal appendage as an active ingredient.
(14) The cranial nerve regeneration promoter according to (13), wherein the tissue cells derived from fetal appendage are umbilical cord cells, placental cells, egg membrane cells, chorionic cells, amniotic cells or a combination thereof.
(15) The cranial nerve regeneration promoter according to (13) or (14), wherein the tissue cells derived from fetal appendage are Walton colloid-derived mesenchymal cells, amnionic mesenchymal cells or chorionic mesenchymal cells, or a combination thereof.
(16) The cranial nerve regeneration promoter according to any one of (13) to (15), wherein the cranial nerve regeneration promoter is a cranial neural progenitor cell proliferating/survival promoter, a cranial neural progenitor cell homing promoter, a cranial neural progenitor cell differentiation/maturation promoter, or a combination thereof.
(17) The cranial nerve regeneration promoter according to any one of (13) to (16), wherein the culture supernatant comprises cytokines and chemokines.
(18) The cranial nerve regeneration promoter according to any one of (13) to (17), comprising IL-6, CXCL1, CXCL7, CXCL8 and CCL2.
(19) The cranial nerve regeneration promoter according to any one of (13) to (18), wherein the culture supernatant is obtained by culturing tissue cells derived from fetal appendage for 1 to 3 days.
(20) The cranial nerve regeneration promoter according to any one of (13) to (19), wherein the density of postpartum tissue is 5 × 10⁴ to 5 × 10⁶/mL at the start of culturing.
(21) The cranial nerve regeneration promoter according to any one of (13) to (20), wherein the cranial nerve regeneration occurs with non-hereditary cranial nerve disorder.
(22) The cranial nerve regeneration promoter according to any one of (13) to (21), wherein the cranial nerve regeneration occurs with dyskinesia (levodopa-induced dyskinesia, chronic or tardive dyskinesia, or orofacial dyskinesia), restless leg syndrome (drug-induced or idiopathic), drug-induced dystonia, chorea (Huntington's disease, toxin-induced chorea, Sydenham chorea, chorea of pregnancy, Wilson's disease, drug-induced chorea or metabolic or endocrine chorea), facial spasm (such as mobile, phonetic, simple, complex or Tourette's syndrome), dystonia (such as acute, systemic, localized, segmental, sexual, neutral, psychogenic or acute dystonic reaction), Sodemytopic Parkinson's disease, stereotypic movement disorder (such as autism or heredity or childhood-related movement disorder), obsessive-compulsive disorder, narcolepsy (such as cataplexy), transmissible spongiform encephalopathy (such as Creutzfeldt-Jakob disease or kuru), dementia (such as Alzheimer's, Lewy body dementia, vascular dementia, Pick's disease or alcoholic dementia), neuroacanthocytosis, seizure or convulsion, athetosis (such as Huntington's disease, respiratory arrest, neonatal jaundice- or stroke-related), or cerebral palsy.
(23) A method for producing a cranial nerve regeneration promoter according to any one of (13) to (22), the method including (a) a step of culturing tissue cells derived from fetal appendage, and (b) a step of recovering the culture supernatant of the tissue cells derived from fetal appendage.
(24) A pharmaceutical composition comprising a cranial nerve regeneration promoter according to any one of (13) to (22).
(25) The pharmaceutical composition according to (24), which is a lyophilized preparation.

### [ADVANTAGEOUS EFFECTS OF INVENTION]

According to the invention it is possible to provide a therapeutic agent for cranial nerve disorder, comprising a culture supernatant of tissue cells derived from fetal appendage. The formulation can be combined with numerous donor cell culture supernatants to produce a homogeneous cranial nerve disorder therapeutic agent, and can be more easily produced, stored and administered compared to cell-containing formulations.

### BRIEF DESCRIPTION OF DRAWINGS

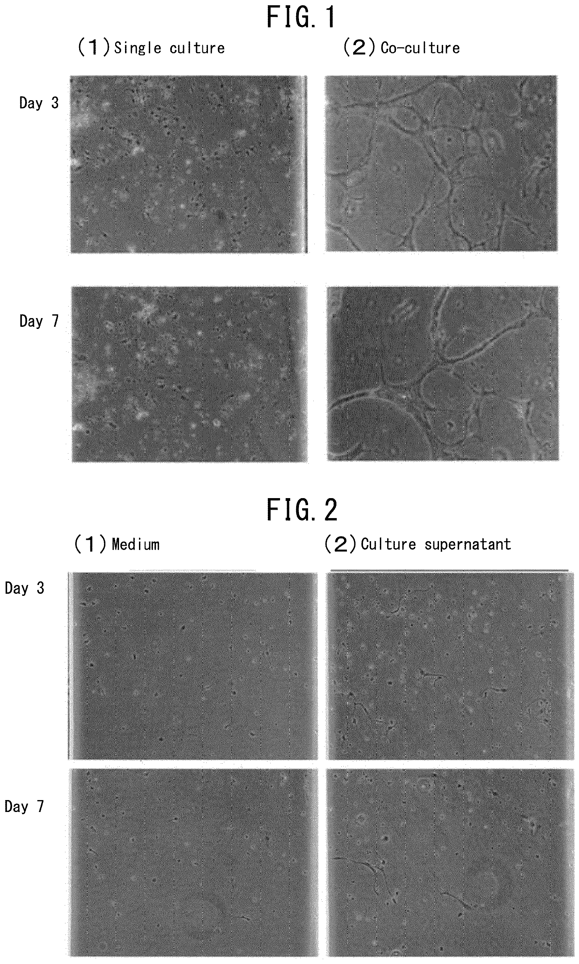

Fig. 1 shows nerve regeneration by co-culturing of Walton colloid-derived mesenchymal cells and neural progenitor cells. With co-culturing (2), proliferation and survival of neural progenitor cells was maintained and movement (migration) and differentiation/maturation were accelerated on the 3rd day and 7th day of culturing, compared to single culture (1).
Fig. 2 shows nerve regeneration *(in vitro)* by Walton colloid-derived mesenchymal cell culture supernatant. With addition of culture supernatant (2), survival of neural progenitor cells was maintained and movement (migration) and differentiation/maturation were accelerated on the 3rd day and 7th day of culturing, compared to addition of medium alone (1).

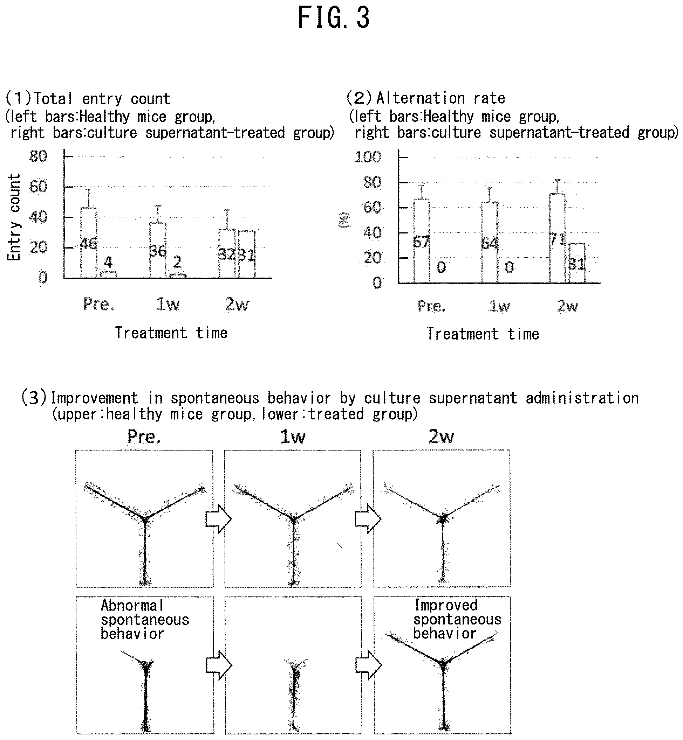

Fig. 3 shows treatment with Walton colloid-derived mesenchymal cell culture supernatant (1). When the culture supernatant was administered to hypoxic pediatric paralysis model mice (left bars), improvement was observed in (1) total entry count and (2) alternation rate in a Y-maze test, compared to administration of medium alone (right bars). Shown is the pattern of spontaneous behavior in the Y-maze test, by normal mice (upper) and mice administered culture supernatant (lower) (3).

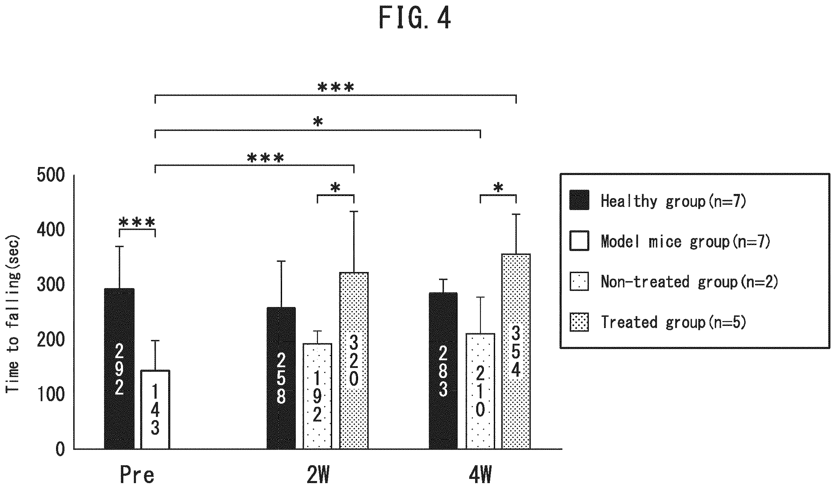

Fig. 4 shows treatment with Walton colloid-derived mesenchymal cell culture supernatant (2). Before treatment (Pre), the hypoxic pediatric paralysis model mice group (right bar) had lower motor function in a Rotarod test, compared to the healthy group (left bar). During 2 weeks following treatment (2W) and subsequent 2 weeks without drug (4W), compared to the control group (middle bars), the treatment group of hypoxic pediatric paralysis model mice administered culture supernatant (right bars) significantly recovered motor function in the Rotarod test (p < 0.05, Tukey statistical test), to a level equivalent to the healthy group (left bars).

### DESCRIPTION OF EMBODIMENTS

The invention will now be described in greater detail by concrete embodiments. However, the invention is not restricted to the described embodiments and may be carried out in any form within a range that is not outside of the gist of the invention.

All of the references including patent publications, patent applications and non-patent publications cited throughout the present disclosure are invoked in their entirety and are incorporated herein for all purposes.

For the purpose of the present disclosure, the word "to" when used between numerical values indicates a range that is above and including the lower value and is below and including the upper value.

### 1. Tissue derived from fetal appendage

The phrase "tissue derived from fetal appendage" (or "postpartum tissue") as used herein means a group of tissues extracted during neonatal delivery, and includes the umbilical cord, the egg membrane (amnion, chorion and decidua), and the placenta. The tissue derived from fetal appendage according to the invention is not particularly restricted so long as it is tissue derived from fetal appendage that has been harvested from a mammal, and it may be tissue derived from fetal appendage of a primate mammal, for example. It is more preferably tissue derived from fetal appendage of a human.

The term "umbilical cord" as used herein means the white tubular tissue that connects the fetus and placenta, but does not include the placenta. The term "Walton colloid", as an essential part of the umbilical cord, refers to connective tissue from the extraembryonic mesoderm, which protects the umbilical cord by covering two navel arteries and the navel vein. The umbilical cord of the invention is not particularly restricted so long as it is harvested from a mammal, and it may be a primate mammalian umbilical cord, for example. It is more preferably a human umbilical cord.

The term "egg membrane" as used herein means a membrane that encompasses amnionic fluid. An egg membrane is not a single membrane but rather is composed of 3 layers: the "decidua", "chorion" and "amnion" that develop from the endometrium. The egg membrane of the invention is not particularly restricted so long as it is an egg membrane harvested from a mammal, and it may be a primate mammalian egg membrane, for example. It is more preferably a human egg membrane. The amnion as the inner layer of the egg membrane, the chorion as the intermediate layer and the decidua as the outer layer may also be used each separately, or the membrane tissues may be used in combination.

The term "placenta" as used herein refers to placental tissue that can be obtained from the body of a mother after delivery. The placenta can be easily extracted after delivery, but it is preferred to use human placenta obtained after harvesting and separation or from umbilical cord blood from a cord blood bank, with informed consent. The placenta is preferably used after examining details of test results for infectious disease, for example. The placenta of the invention is not particularly restricted so long as it is harvested from a mammal, and it may be a primate mammalian placenta, for example. It is more preferably a human placenta.

The term "tissue cells derived from fetal appendage" (or "postpartum tissue") as used herein refers to cells obtained by physical and/or enzymatic treatment of tissue derived from a fetal appendage, and it differs from specially isolated stem cells. The tissue cells derived from fetal appendage differ from different stem cell types that are derived from umbilical cord blood or tissue derived from fetal appendage, in that they do not require multigenerational subculturing or detailed cell fractionation and are thus easy to prepare.

### 2. Recovery and treatment of tissue derived from fetal appendage

Tissue derived from fetal appendage is generally recovered immediately after birth. According to a preferred embodiment, the tissue derived from fetal appendage is recovered from a donor after informed consent, or via a cord blood bank. Preferably, the donor medical record and test results for infectious disease are associated with the tissue derived from fetal appendage. Such a medical record is used to restrict the use of the tissue derived from fetal appendage, or tissue cells derived from fetal appendage harvested from the tissue.

Before recovering the tissue cells derived from fetal appendage, the blood including umbilical cord blood and placental blood is preferably removed and/or washed off. According to one specific embodiment, the umbilical cord blood in the placenta is collected. A conventional umbilical cord blood-collecting process may be employed for this purpose. Typically, a needle or cannula is used under flow to collect the blood from the placenta (see Anderson, US Patent No. 5,372,581; or Hessel et al., US Patent No. 5,415,665, for example). A needle or cannula can usually be placed in the navel vein and the placenta may be lightly massaged to facilitate outflow of the umbilical cord blood from the placenta. The placenta is preferably bled by flow without any further procedures in order to minimize destruction of the tissue during recovery of the umbilical cord blood.

After the mammalian tissue derived from fetal appendage, or a portion thereof, has been recovered and prepared as described above, it may be treated by a method known in the relevant technical field, such as perfusion, washing, chopping, fracture or adhesion onto a culture vessel, and for example, after chopping it may be digested with one or more tissue-degrading enzymes or adhered onto a culture vessel to obtain tissue cells derived from fetal appendage.

According to one embodiment, the cells may be recovered from tissue derived from a mammalian fetal appendage by physical destruction of all or a portion of an organ. For example, tissue derived from fetal appendage or a portion thereof may be crushed, sheared, divided, diced, chopped, finely crushed or passed through a mesh. Typically, the tissue derived from fetal appendage is crushed while immersed in a solution that does not impair survival of the cells, such as medium or physiological saline. The tissue may then be cultured to obtain an aggregate of adhering tissue cells derived from the fetal appendage.

The tissue derived from fetal appendage may also be cut into separate different tissues before physical destruction and/or enzyme digestion and cell recovery. For example, it may be cut to obtain all or a portion of an egg membrane such as amnion or chorion, umbilical cord, placental lobe or any combination thereof. The tissue cells derived from fetal appendage are preferably obtained from amnion, chorion, umbilical cord (such as Walton colloid) or any combination thereof. The tissue cells derived from fetal appendage can typically be obtained by crushing small masses of tissue derived from fetal appendage, such as masses of tissue derived from fetal appendage that are about 1, 2, 3, 4, 5, 6, 7, 8, 9, 10, 20, 30, 40, 50, 60, 70, 80, 90, 100, 200, 300, 400, 500, 600, 700, 800, 900 or 1000 cubic millimeters in volume. Any physical crushing method may be used under conditions in which the crushing method allows survival of many, more preferably most and even more preferably at least 60%, 70%, 80%, 90%, 95%, 98% or 99% of the cells in the tissue derived from fetal appendage, as determined by measurement using Trypan blue dye exclusion testing, for example.

According to another specific embodiment, the tissue cells derived from fetal appendage are recovered by physical crushing of tissue derived from fetal appendage, in which case the physical crushing may be accompanied by enzyme digestion using one or more tissue-digesting enzymes. Enzymes that can be used for digestion of tissue derived from fetal appendage include papain, deoxyribonuclease, serine proteases, trypsin, chymotrypsin, collagenase, dispase and elastase. Serine proteases can be inhibited by alpha-2-microglobulin in serum, while serum also contains large amounts of substrate proteins, and therefore the medium used for digestion is usually serum-free. EDTA and/or DNase may also be used during digestion with enzymes to increase the cell recovery efficiency.

Tissue-digesting enzymes may also be used in any desired combinations. A typical concentration for digestion using trypsin is about 0.05% to 2% trypsin, such as about 0.05% trypsin. Proteases may be used in combinations, such as two or more proteases in the same digestion reaction, and they may be used simultaneously to cut out the tissue cells derived from fetal appendage. According to one embodiment, for example, placenta or a portion thereof is first digested using a suitable amount of collagenase I at about 1 to 2 mg/ml for 30 minutes, for example, and is then digested using trypsin at a concentration of about 0.25% for 10 minutes, for example, at 37°C. A serine protease is preferably used successively after other enzymes have been used.

After digestion, the digested product is washed with serum-containing culture medium, for example, and the washed cells are seeded in a culture vessel. The cells are then isolated by differential adhesion to prepare mesenchymal cells. It is thus possible to prepare umbilical cord Walton colloid-derived mesenchymal cells, egg membrane amnionic mesenchymal cells, egg membrane chorionic mesenchymal cells or placental mesenchymal cells, for example. Before preparing culture supernatant, the mesenchymal cells may be subcultured in serum-containing culture medium several times, and preferably up to 10, 9, 8, 7, 6, 5, 4 or 3 times. The tissue cells derived from fetal appendage according to the present disclosure can be easily prepared without a special step of isolating stem cells.

### 3. Preparation of culture supernatant

The "culturing" of the tissue cells derived from fetal appendage may be carried out by a common method such as the following, for example. The tissue cells derived from fetal appendage are added to basal medium in a culture vessel also containing sodium, potassium, calcium, magnesium, phosphorus, chlorine, amino acids, vitamins, hormones, cytokines, antibiotics, fatty acids, sugars or other chemical components, or biological components such as serum, as necessary for the purpose, and cultured in a CO₂ incubator with adjustment to a culturing temperature of 30°C to 40°C and preferably 37°C, and a carbon dioxide gas concentration of 0.03% to 40%, preferably 5 to 10% and even more preferably 5%. The oxygen concentration may be the same concentration as air, or it may be a lower concentration (1 to 20%). For example, the oxygen concentration may be 1%, 2%, 3%, 4%, 5%, 6%, 7%, 8%, 9%, 10%, 11%, 12%, 13%, 14%, 15%, 16%, 17%, 18%, 19% or 20%. The culturing period may be from a short period of several hours up to 1 day, 1.5 days, 2 days, 3 days, 5 days or 7 days.

The medium (culture solution) used may be any one that does not inhibit proliferation, maintenance and survival of the tissue cells derived from fetal appendage, or secretion of proteins. The basal medium used may be Dulbecco's Modified Eagle Medium (DMEM), or Iscove's Modified Dulbecco's Medium (IMDM), Ham F12 medium (HamF12) or RPMI 1640 medium. Two or more basal media may also be used in combination. An example of mixed medium is an equivolume mixture of IMDM and HamF12 medium (for example, IMDM/HamF12). Examples of components to be added to the medium include serum (such as fetal bovine serum, human serum, horse serum or sheep serum), serum substitute (such as Knockout serum replacement), conditioned medium, bovine serum albumin, human serum albumin, antibiotics, vitamins, deep sea water or minerals.

For the purpose of the present disclosure, the medium for tissue cells derived from fetal appendage to be used for preparation of a cranial nerve disorder therapeutic agent preferably contains no serum. Using serum-free medium can lower the risk of infection by prions and the like and prevent contamination by heteroproteins such as bovine serum albumin, thereby increasing the safety of the cranial nerve disorder therapeutic agent. For example, culturing tissue cells derived from fetal appendage in medium without serum (serum-free medium) allows culture supernatant to be prepared that is free of serum components.

The medium used to prepare the cranial nerve disorder therapeutic agent of the disclosure may include deep sea water. The term "deep sea water" generally refers to seawater at a location greater than a depth of 200 meters, which around the region of Greenland is the origin of vertically sinking sea currents on a global scale known as "plumes" which are produced by difference in salt concentration, and consists of seawater that has been moving over the Earth for many centuries without ever contacting the atmosphere. Deep sea water is virtually shielded from sunlight rays and is in a low temperature high-pressure state, free of bacteria and rich in inorganic nutrient salts (minerals). By including deep sea water it is possible to adjust the amount of secretory proteins in culture supernatant and to prepare a more highly active cranial nerve disorder therapeutic agent than when deep sea water is not included. The concentration (v/v) of deep sea water in the medium may be, for example, 1%, 2%, 3%, 4%, 5%, 6%, 7%, 8%, 9%, 10%, 15%, 20%, 25%, 30%, 40%, 50%, 60%, 70% or 80% or greater, or it may be 90%, 80%, 70%, 60%, 50%, 45%, 40%, 35%, 30%, 25%, 20%, 15% or 10% or lower, such as in the range of 1 to 85%, 5 to 85%, 10% to 85%, 15% to 85%, 20% to 85%, 25% to 85%, 30% to 85%, 40% to 85%, 50% to 85%, 60% to 85%, 70% to 85%, 80% to 85%, 1 to 75%, 5 to 75%, 10% to 75%, 15% to 75%, 20% to 75%, 25% to 75%, 30% to 75%, 40% to 75%, 50% to 75%, 60% to 75%, 70% to 75%, 1 to 45%, 5 to 45%, 10% to 45%, 15% to 45%, 20% to 45%, 25% to 45%, 30% to 45%, 40% to 45%, 1 to 10% or 5 to 10%, or it may be 73% or 81%.

The culturing time for obtaining the culture supernatant of tissue cells derived from fetal appendage may be, for example, 5 hours to 7 days, 1 day to 6 days, 1 day to 5 days, 1 day to 4 days, 1 day to 3 days or 1 day to 2 days, or it may be 0.5 day, 1 day, 2 days, 3 days, 4 days, 5 days, 6 days or 7 days. The culturing temperature may be 36°C to 38°C, such as 37°C, and the CO₂ concentration may be 4 to 6%, such as 5%, for example. The culturing may be three-dimensional culturing under non-adhesive conditions, for example, or suspension culturing (such as dispersion culturing or aggregated suspension culturing).

The density of tissue cells derived from fetal appendage at the start of culturing, to obtain a culture supernatant of tissue cells derived from fetal appendage, may be 1 × 10⁴ to 1 × 10⁷/mL, 5 × 10⁴ to 5 × 10⁶/mL, 1 × 10⁵ to 5 × 10⁶/mL, 2 × 10⁵ to 5 × 10⁶/mL, 5 × 10⁵ to 5 × 10⁶/mL or 1 × 10⁶ to 5 × 10⁶/mL, such as 2 × 10⁵/mL, 3 × 10⁵/mL, 4 × 10⁵/mL, 5 × 10⁵/mL, 6 × 10⁵/mL, 7 × 10⁵/mL, 8 × 10⁵/mL, 9 × 10⁵/mL, 1 × 10⁶/mL, 2 × 10⁶/mL, 3 × 10⁶/mL, 4 × 10⁶/mL, 5 × 10⁶/mL, 6 × 10⁶/mL, 7 × 10⁶/mL, 8 × 10⁶/mL, 7 × 10⁶/mL, 8 × 10⁶/mL, 9 × 10⁶/mL or 1 × 10⁷/mL.

After culturing, the cell components may be separated and removed to obtain the culture supernatant of tissue cells derived from fetal appendage. According to this disclosure, "culture supernatant" refers not only to the supernatant after separation and removal of the cell components from the culture solution, but also to culture supernatant that has been subjected to various types of treatment (such as centrifugation, concentration, solvent exchanged, dialysis, freezing, drying, freeze-drying, dilution, desalting and storage). Details regarding treatment methods for the culture supernatant are described below. Culture supernatant according to the disclosure does not contain the cell components. The culture supernatant of the disclosure therefore does not contain the tissue cells derived from fetal appendage used for culturing, and is not a composition for cellular medicine.

The tissue cells derived from fetal appendage of the disclosure secrete cytokines, chemokines and growth factors into the medium. Proteins present in the culture supernatant include Interleukin(IL)-6, C-X-C Motif Chemokine Ligand (CXCL)1, CXCL7, CXCL8 (IL-8) and C-C Motif Chemokine Ligand (CCL)2 (MCP-1), which are important for cranial nerve regeneration.

For the purpose of the disclosure, the culture supernatant of tissue cells derived from fetal appendage may include one or more of the following proteins:
cytokines such as the TNF Superfamily Member 14 (LIGHT), Interleukin(IL)-1α, IL-1b, IL-2, IL-3, IL-4, IL-5, IL-7, IL-10, IL-12p40/70, IL-13, IL-15, IL-16, Interferon (IFN)-y, Tumor Necrosis Factor (TNF)-α and TNF-β;
chemokines such as C-X-C Motif Chemokine Ligand (CXCL)1/2/3 (GROα/β/γ), CXCL6, CXCL9 (MIG), CXCL10 (IP-10), CXCL12 (SDF-1), CXCL13, C-C Motif Chemokine Ligand (CCL)1, CCL4, CCL5, CCL7, CCL8, CCL11, CCL13, CCL15, CCL17, CCL18, CCL20 (LARC), CCL22, CCL23, CCL24, CCL26 and C-X3-C Motif Chemokine Ligand (CX3CL)1 (FKN);
growth factor/related factors such as Angiogenin, Brain Derived Neurotrophic Factor (BDNF), Epidermal Growth Factor (EGF), Fibroblast Growth Factor (FGF)-4, FGF-6, FGF-7, FGF-9, Fms Related Receptor Tyrosine Kinase-3 Ligand (FLT-3L), Glial Cell Derived Neurotrophic Factor (GDNF), Granulocyte-Macrophage Colony-Stimulating Factor (GM-CSF), Granulocyte Colony-Stimulating Factor (G-CSF), Hepatocyte Growth Factor (HGF), Insulin Like Growth Factor (IGF)-1, Insulin Like Growth Factor Binding Protein (IGFBP)-2, IGFBP-1, IGFBP-4, IGFBP-3, Platelet Derived Growth Factor (PDGF)-BB, Placental Growth Factor (PLGF), Transforming Growth Factor (TGF)-β1, TGF-β2, TGF-β3, Thrombopoietin (TPO), Stem Cell Factor (SCF), Macrophage Colony Stimulating Factor (M-CSF), Neurotrophin (NT)-3, NT-4 and Vascular Endothelial Growth Factor (VEGF)-A; and
other mediators such as Leptin, Leukemia Inhibitory Factor (LIF), Macrophage Migration Inhibitory Factor (MIF), Osteopontin (OPN), Osteoprotegerin (OPG), Oncostatin M (OSM), Tissue Inhibitor of Metalloproteinases (TIMP)-1 and TIMP-2.

Factors that may be included in the culture supernatant of tissue cells derived from fetal appendage of the disclosure are important for nerve regeneration for the following reasons. The culture supernatant of the disclosure includes all or some of these factors, that are suitable for brain or central nerve regeneration (Boruczkowski et al., Int. J. Mol. Sci. 2019 20, 2433; Watson et al., Neuroscience Letters 2020 715, 134533; Wang et al., Stem Cell Research & Therapy 2017 8,26; Baba et al., PLOS ONE 2019 14(9)e0221111; Wang et al., Acta Medica Okayama 2012 66(6) 429).
a) Proliferation and survival of neural progenitor cells: BDNF, CCL5, CXCL1, CXCL7, CXCL8, CX3CL1, EGF, FGF-9, G-CSF, IGF-1, IL-6, OPN.
b) Differentiation and maturation of neural progenitor cells: BDNF, CCL11, CXCL1, CXCL7, CXCL9, CXCL12, CX3CL1, FGF-4, FGF-9, GDNF, GM-CSF, IL-1β, LIF, OPN, SCF, TGF-β3.
c) Migration of neural progenitor cells: CCL2, CCL11, CCL20, CXCL1, CXCL7, CXCL8, CXCL12, EGF, IGF-1.
d) Neurotrophy: GDNF, NGF, NT-4/5.
e) Activation of astrocytes: BDNF, IFN-y, IL-1α, IL-1β.
f) Activation of microglial cells: CCL17, CXCL10, Leptin.
g) Regeneration of oligodendrocytes: CXCL1, CXCL7, CXCL8, CXCL12, CX3CL1, EGF, M-CSF, NT-4, IGFBP-4.
h) Vascularization: angiogenin, CCL2, CXCL5, CXCL6, CXCL7, CXCL8, Groα/β/γ, HGF, IGF-1, IL-6, VEGF, SCF.
i) Anti-inflammation: CCL4, IL-10, IL-13, TGF-β2.

According to the disclosure, the culture supernatant of tissue cells derived from fetal appendage which is a mixture of cytokines and chemokines (and growth factors) can be used as a portion of the fetal appendage-derived tissue cell culture supernatant, or as a mixture of cytokines and chemokines (and growth factors) isolated from the fetal appendage-derived tissue cell culture supernatant. A mixture of cytokines and chemokines (and growth factors) isolated from the fetal appendage-derived tissue cell culture supernatant may have some of the cytokines and chemokines (and growth factors) replaced by one or more corresponding gene-recombinant cytokines and chemokines (and growth factors), or corresponding gene-recombinant cytokines and chemokines (and growth factors) may be added.

### 4. Cranial nerve disorder therapeutic agent

By comprising a culture supernatant of tissue cells derived from fetal appendage, the cranial nerve disorder therapeutic agent of the disclosure exhibits an effect of regenerating nerve tissue in cranial nerve disorder. The level of the effect is comparable in full or in part to the conventional effect of grafting umbilical cord blood stem cells. Surprisingly, the cranial nerve disorder therapeutic agent of the disclosure exhibits a general nerve regeneration effect across a wide range of cranial nerve tissues, by promoting proliferation of neural progenitor cells in the subventricular zone, homing to sites of cranial nerve disorder, and differentiation/maturation of neurons at sites of cranial nerve damage. The cranial nerve disorder therapeutic agent of the disclosure therefore is not limited to any specific cranial nerve disorder, and it can be used as a cranial nerve regeneration promoter for a wide range of cranial nerve disorder, to treat and alleviate the symptoms of cranial nerve disorder. The cranial nerve disorder therapeutic agent of the invention can effectively inhibit progression of cranial nerve disorder by administration before cranial nerve disorder, even in unknown stages of disease that may lead to such cranial nerve disorder. By using the cranial nerve disorder therapeutic agent of the disclosure in a habitual manner it is possible to treat a variety of cranial nerve disorders before onset and thus prevent, delay or alleviate their onset. The term "treat" as used herein includes not only treatment for complete curing of symptoms or anomalies associated with cranial nerve disorder, but also alleviation of symptoms or anomalies that do not result in complete curing, or treatment that delays them compared to lack of treatment, as well as prevention, delay or alleviation of onset.

Since the cranial nerve disorder therapeutic agent of the disclosure promotes nerve regeneration, it can be used for a wide range of types of cranial nerve disorders. Non-hereditary cranial nerve disorder is an example of cranial nerve disorder. Without being limitative, the cranial nerve disorder therapeutic agent of the disclosure can be applied for dyskinesia (levodopa-induced dyskinesia, chronic or tardive dyskinesia, or orofacial dyskinesia), restless leg syndrome (drug-induced or idiopathic), drug-induced dystonia, chorea (Huntington's disease, toxin-induced chorea, Sydenham chorea, chorea of pregnancy, Wilson's disease, drug-induced chorea or metabolic or endocrine chorea), facial spasm (such as mobile, phonetic, simple, complex or Tourette's syndrome), dystonia (such as acute, systemic, localized, segmental, sexual, neutral, psychogenic or acute dystonic reaction), Sodemytopic Parkinson's disease, stereotypic movement disorder (such as autism or heredity or childhood-related movement disorder), obsessive-compulsive disorder, narcolepsy (such as cataplexy), transmissible spongiform encephalopathy (such as Creutzfeldt-Jakob disease or kuru), dementia (such as Alzheimer's, Lewy body dementia, vascular dementia, Pick's disease or alcoholic dementia), neuroacanthocytosis, seizure or convulsion, athetosis (such as Huntington's disease, respiratory arrest, neonatal jaundice- or stroke-related), or cerebral palsy.

According to one embodiment, a brain disorder, such as signs or symptoms of cerebral palsy, to be treated by the cranial nerve disorder therapeutic agent of the disclosure is motor function impairment, language or speech dysfunction, ambulation or mobile dysfunction (such as reduced walking speed), dysfunctional muscle strength, dysfunctional muscle tone, abnormal reflex, abnormal coordination, spasticity, involuntary movement, abnormal ambulation, abnormal balance, reduced muscle mass, impaired skeletal development or impaired muscle development. According to one embodiment, the brain disorder, such as signs or symptoms of cerebral palsy, to be treated by the cranial nerve disorder therapeutic agent of the disclosure is hand strength impairment, manual dexterity impairment, walking speed impairment or gait disturbance. According to one embodiment, the brain disorder, such as signs or symptoms of cerebral palsy, to be treated by the cranial nerve disorder therapeutic agent of the disclosure is sensory motor disturbance or sensory motor function impairment, including but not limited to ataxia, systemic control disorder, impaired coordination or balance, impaired body sensation, impaired proprioceptive sensation, impaired endurance, impaired hand function, impaired hand strength, loss or impairment of fine hand coordination, hyperreflexia, impaired grip strength, reduced muscle strength, impaired muscle tone, impaired range of motion, spasticity, impaired or weakened strength, tremor, impaired limb function, upper extremity dysfunction, lower extremity dysfunction, impaired lower extremity muscle strength, gait disturbance (such as reduced walking speed), impaired ability to stand, impaired speech (such as stammering), impaired jaw function, chewing impairment, temporomandibular joint impairment, impaired dexterity, reflection, or impairment of any other sensory motor functions mentioned herein or known in the technical field.

The target of administration of the cranial nerve disorder therapeutic agent will typically be a human patient with damage to target tissue, but the agent may also be applied to a mammal other than a human (including pet animals, livestock and experimental animals, and specifically mice, rats, guinea pigs, hamsters, monkeys, cows, pigs, goats, sheep, dogs or cats).

There are no particular restrictions on the site where the cranial nerve disorder has occurred. The diseased site may be all or part of the brain, the brain including both the forebrain and brain stem, and the forebrain including the cerebrum and diencephalon, and the brain stem including the midbrain and hindbrain. The cerebrum includes the olfactory brain, amygdala, striatum, hippocampus and cerebral neocortex, and the diencephalon includes the epithalamus, thalamus, hypothalamus, thalamus abdomen, pituitary, pineal body and third cerebral ventricle. The midbrain includes the mesencephalon, cerebral peduncle, preoptic area and cerebral aqueduct, and the hindbrain includes the pons, cerebellum and medulla oblongata. A site of cranial nerve disorder may be any of these sites.

The treatment method using the cranial nerve disorder therapeutic agent of the disclosure includes nasal (intranasal) administration of the fetal appendage-derived tissue cell culture supernatant for repair of the brain disorder site. This treatment method allows low-invasive and effective restoration of regions suffering from injury due to cranial nerve disorder such as cerebral palsy.

### 5. Production method

The disclosure provides a method for producing a cranial nerve disorder therapeutic agent which includes:
(a) a step of culturing tissue cells derived from fetal appendage, and (b) a step of recovering the culture supernatant of the tissue cells derived from fetal appendage. The production method may further include a step of carrying out one or more processes selected from among centrifugation, concentration, solvent exchange, dialysis, freezing, drying, freeze-drying, dilution and desalting of the collected culture supernatant. Including these steps will further facilitate handling, storage and transport of the cranial nerve disorder therapeutic agent. The production method may also include a step of adding additional components to the collected culture supernatant. Addition of other components can alter the physical properties and improve the properties of the composition for treatment of cranial nerve disorder. The production method may also further include a step of extracting tissue derived from fetal appendage and a step of preparing somatic tissue cells derived from fetal appendage, from the tissue derived from fetal appendage. The conditions described for the cranial nerve disorder therapeutic agent of the disclosure all apply for each of these steps and additional components. When the method includes both a step of carrying out one or more processes selected from among centrifugation, concentration, solvent exchange, dialysis, freezing, drying, freeze-drying, dilution and desalting of the collected culture supernatant, and a step of adding additional components to the collected culture supernatant, the steps may be carried out in any order, or when possible they may be carried out simultaneously in parallel.

In step (b), the fetal appendage-derived tissue cell culture supernatant is collected. The culture solution may be collected by drawing with a dropper or pipette, for example. The collected culture supernatant is used as an active ingredient of the cranial nerve disorder therapeutic agent of the disclosure, either directly or after being treated by one or more treatments. The treatment may be, for example, centrifugation, concentration, solvent exchange, dialysis, formulation, freezing, drying, freeze-drying, dilution, desalting and storage (such as 4°C or -80°C). The culture supernatant of tissue cells derived from fetal appendage functions as expected even without complex advanced purification. The cranial nerve disorder therapeutic agent of the disclosure can therefore be prepared by a simple procedure. Eliminating the need for complex purification is advantageous because it avoids the reduction in activity that occurs with purification.

The fetal appendage-derived tissue cell culture supernatant of the disclosure may also be derived from tissue cells derived from fetal appendage taken from multiple donors. Specifically, tissue cells derived from fetal appendage taken from multiple donors may be combined and cultured, and the resulting culture supernatant collected, or alternatively the culture supernatants of tissue cells derived from fetal appendages taken from individual donors may be combined, and then subjected to one or more treatments (centrifugation, concentration, solvent exchange, dialysis, formulation, freezing, drying, freeze-drying, dilution, desalting or storage), subsequently combining the culture supernatants of tissue cells derived from fetal appendages derived from the individual donors. However, since the fetal appendage-derived tissue cell culture supernatant of the disclosure has a risk of infection by contaminating viruses and the like, it is not preferred to mix and culture tissue cells derived from fetal appendages from numerous donors. A culture supernatant of tissue cells derived from fetal appendages from multiple donors is advantageous in that it can be produced as a homogeneous cranial nerve disorder therapeutic agent.

### <Method of concentrating fetal appendage-derived tissue cell culture supernatant>

The composition for treatment of cranial nerve disorder of the disclosure may be a formulated preparation. The method of concentrating the fetal appendage-derived tissue cell culture supernatant for formulation may be a method commonly used for concentration of culture supernatants. The following two methods are examples of concentration methods.

### 1) Spin column concentration

The culture supernatant is concentrated using Amicon Ultra Centrifugal Filter Units-10K (Millipore). The specific procedure is as follows.
(i) The culture supernatant (maximum 15 mL) is loaded into Amicon Ultra Centrifugal Filter Units-10K and centrifuged at × 4000 g for about 60 minutes for concentration to 200 µL.
(ii) The culture supernatant and an equivolume of sterilized PBS are loaded into the tube, and centrifugation is repeated at × 4000 g for about 60 minutes, replacing the base solution with the PBS.
(iii) A 200 µL portion of the obtained solution is collected in a micro test tube as the concentrated fetal appendage-derived tissue cell culture supernatant.

### 2) Ethanol precipitation concentration

The culture supernatant is concentrated by ethanol precipitation. The specific procedure is as follows.
(i) A 45 mL portion of 100% ethanol is added to and mixed with 5 mL of culture supernatant, and the mixture is allowed to stand at -20°C for 60 minutes.
(ii) Centrifugation is carried out at 4°C × 15,000 g for 15 minutes.
(iii) The supernatant is removed, 10 mL of 90% ethanol is added and the mixture is thoroughly stirred.
(iv) Centrifugation is carried out at 4°C × 15,000 g for 5 minutes.
(v) The supernatant is removed, and the obtained pellet is dissolved in 500 µL of sterilized water and collected in a micro test tube as the concentrated fetal appendage-derived tissue cell culture supernatant.

### <Method of freeze-drying fetal appendage-derived tissue cell culture supernatant>

The fetal appendage-derived tissue cell culture supernatant for the cranial nerve disorder therapeutic agent of the disclosure may be freeze-dried. This will help provide more satisfactory storage stability. The method of freeze-drying the fetal appendage-derived tissue cell culture supernatant may be a method commonly used for freeze-drying of culture supernatants. The following method is an example of a freeze-drying method. The freeze-dried culture supernatant may be used as a powder formulation or it may be reconstituted for use in a suitable solvent such as water.
(i) The fetal appendage-derived tissue cell culture supernatant or concentrated fetal appendage-derived tissue cell culture supernatant obtained as described above is frozen at -80°C for a period of 2 to 12 hours.
(ii) After freezing, the sample tube cover is opened and the tube is set in a freezing dryer.
(iii) Freeze-drying is carried out for 1 to 2 days.
(iv) The obtained sample is used as a freeze-dried fetal appendage-derived tissue cell culture supernatant (storable at -80°C).

### 6. Pharmaceutical composition

The cranial nerve disorder therapeutic agent of the disclosure may also include other components for a pharmaceutical composition, for the purpose of supporting the expected curative effect in light of the condition of the subject to which is to be applied. Examples of additional components include the following.

### (i) Bioabsorbable materials

Hyaluronic acid, collagen and fibrinogen (such as BOLHEAL^{™}) may be used as organic bioabsorbable materials.

### (ii) Gelling materials

Gelling materials are preferably highly biocompatible materials, such as hyaluronic acid, collagen or fibrin paste. Various types of hyaluronic acid or collagen may be selected for use, but they are preferably selected as suited for the purpose of use (the target tissue) of the cranial nerve disorder therapeutic agent of the disclosure. The collagen used is preferably soluble (acid-soluble collagen, alkali-soluble collagen or enzyme-soluble collagen).

### (iii) Other components

Other pharmaceutically acceptable components (for example, carriers, excipients, disintegrators, buffering agents, emulsifying agents, suspending agents, soothing agents, stabilizers, preservatives, antiseptic agents, physiological saline and the like) may also be added. Excipients include lactose, starch, sorbitol, D-mannitol and saccharose. Disintegrators that may be used include starches, carboxymethyl cellulose and calcium carbonate. Buffering agents that may be used include phosphates, citrates and acetates. Emulsifying agents that may be used include gum arabic, sodium alginate and tragacanth. Suspending agents that may be used include glycerin monostearate, aluminum monostearate, methyl cellulose, carboxymethyl cellulose, hydroxymethyl cellulose and sodium lauryl sulfate. Soothing agents that may be used include benzyl alcohol, chlorobutanol and sorbitol. Stabilizers that may be used include propylene glycol and ascorbic acid. Preservatives that may be used include phenol, benzalkonium chloride, benzyl alcohol, chlorobutanol and methylparaben. Antiseptic agents that may be used include benzalkonium chloride, paraoxybenzoic acid and chlorobutanol. Antibiotics, pH adjustors, growth factors (such as epidermal growth factor (EGF), nerve growth factor (NGF) and brain-derived neurotrophic factor (BDNF)) may also be added.

The final form of the pharmaceutical composition of the disclosure is not particularly restricted. Examples of dosage forms include liquid forms (liquids and gels) and solid forms (including lyophilized preparations such as powders, fine grains and granules). The pharmaceutical composition of the disclosure may also be in a form suited for inhalation, in which case it may be in a liquid form that is dispersible as a mist by a nebulizer or diffuser. The site of cranial nerve disorder is the brain, and in light of the presence of the blood brain barrier, the composition for treatment of cranial nerve disorder according to the disclosure is preferably in a form that can be administered intranasally, intracerebroventricularly or intrathecally. For example, it may be administered intranasally in the form of a spray or powder. From the viewpoint of prior preparation and storage, the culture supernatant of tissue cells derived from fetal appendage is more advantageous than using tissue cells derived from fetal appendage or stem cells derived from fetal appendage, and may therefore be considered especially suitable for treatment of acute or subacute cranial nerve disorder. The culture supernatant of tissue cells derived from fetal appendage is also highly useful from the viewpoint of overcoming immunological rejection as well, since it does not contain cell components.

Depending on the embodiment, however, the cranial nerve disorder therapeutic agent of the disclosure may still include tissue cells derived from fetal appendage, stem cells derived from fetal appendage, ES cells, iPS cells or mesenchymal stem cells in addition to the culture supernatant of tissue cells derived from fetal appendage, or it may be used in tandem with such stem cells. Using stem cells in addition may further improve the therapeutic effect.

The present disclosure also provides a method of inhibiting onset of cranial nerve disorder in a subject prior to onset of the cranial nerve disorder, the method comprising administration of the cranial nerve disorder therapeutic agent of the disclosure at a dose effective for preventing onset of the cranial nerve disorder, before onset of the cranial nerve disorder. The subject may be a human, or a mammal other than a human (such as a pet animal, farm animal or experimental animal, and specifically, a mouse, rat, guinea pig, hamster, monkey, cow, pig, goat, sheep, dog or cat). The subject may also be one assessed to be at risk for onset of cranial nerve disorder. Such risk can be assessed by gene diagnosis or genealogical analysis. For example, it has been statistically shown that the presence of specific alleles or SNPs in specific genes are correlated with greater likelihood of specific diseases.

The dose of the cranial nerve disorder therapeutic agent of the disclosure is not limited so long as the expected therapeutic effect is maintained, and it may be 0.1 mL/kg/day to 100 mL/kg/day, 1 mL/kg/day to 100 mL/kg/day or 5 mL/kg/day to 100 mL/kg/day, in terms of the amount of untreated culture supernatant, or 0.1 mg/kg/day to 1000 mg/kg/day or 1 mg/kg/day to 100 mg/kg/day, in terms of protein level of the untreated culture supernatant. The method of administration is also not particularly restricted. The cranial nerve disorder therapeutic agent is preferably administered by parenteral administration, for example, with either systemic or local administration as the method of parenteral administration. Examples of methods of administering the cranial nerve disorder therapeutic agent are not particularly restricted so long as the expected therapeutic effect is maintained, and it may be intravenous administration, intraarterial administration, intraportal administration, intradermal administration, subcutaneous administration, intramuscular administration, intraperitoneal administration, transpulmonary administration (transpulmonary absorption), intracerebroventricular administration, intrathecal administration or nasal administration, for example. Intranasal administration is preferred because it has low invasiveness. Intracerebroventricular administration, intrathecal administration and intranasal administration can ensure passage through the blood brain barrier and are therefore particularly effective for treatment of cranial nerve disorder. The dosing schedule may be once or several times per day, or once every two days, or once every three days. The gender, age, body weight and pathology of the subject may be considered when creating the dosing schedule.

### 7. Cranial nerve regeneration promoter

The fetal appendage-derived tissue cell culture supernatant of the disclosure is a cranial nerve regeneration promoter. The term "cranial nerve regeneration" refers to at least one of many phenomena during the course of development, including nerve repair or nerve development by increase, differentiation and maturation of neural progenitor cells in the brain. Nerve regeneration therefore preferably includes the phenomenon of complete or partial recovery of original nerve function. A publicly known method may be used to confirm that nerve regeneration has been efficiently achieved. For example, a patient or livestock animal having nerve injury and having been administered a nerve regeneration promoter may be compared with a patient or livestock animal having nerve injury and not having been administered a nerve regeneration promoter, and it may be judged that efficient nerve regeneration has been achieved if the patient or livestock animal that has been administered the nerve regeneration promoter has greater recovery in the function of the damaged nerve. Recovery of nerve function can be evaluated based on reaction to stimulation or recovery of motor function, as demonstrated in the Examples below. Recovery of motor function can be examined by a publicly known method, such as a Rotarod test (Oh et al., Exp. Mol. Med. 2018 50(4), 22; Kossatz et al., Front. Pharmacol. 2018 9, 376), a Y-maze test (Miedel et al., J. Vis. Exp. 2017(123); Sarnyai et al., Proc. Natl. Acad. Sci. USA. 2000 97(26), 14731) or a hanging wire test (Aartsma-Rus et al., J. Vis. Exp. 2014(85)).

Nerve regeneration may be by cells derived from defective nerves that are originally from the site to be treated (endogenous cells), or cells that have been grafted together with the nerve regeneration promoter (exogenous cells). Such cells include neurons, neural progenitor cells, embryonic stem cells, induced pluripotent stem cells, mesenchymal stem cells, vascular endothelial cells, vascular endothelial precursor cells and hematopoietic stem cells.

The fetal appendage-derived tissue cell culture supernatant of the disclosure may be a cranial nerve regeneration promoter, or a cranial neural progenitor cell proliferating/survival promoter. The term "cranial neural progenitor cell proliferating/survival promoter" means an agent that promotes proliferation or survival of neural progenitor cells in the brain. A publicly known method may be employed to confirm that proliferation or survival of cranial neural progenitor cells has been effectively achieved. For example, accelerated proliferation or survival of neural progenitor cells in the brain or derived from the brain, or corresponding cells, by addition of the cranial neural progenitor cell proliferating/survival promoter, can be evaluated by observation similar to that described in the Examples described below. Neural progenitor cells harvested from a living body, or cells differentiated from stem cells such as iPS cells, or a cell line (such as A172, B65, C6, KS-1, N2A, PC12, SH-SY5Y, SKN-BE2, T98G, U251, U87 or YH-13) may be used.

The fetal appendage-derived tissue cell culture supernatant of the disclosure is a cranial nerve regeneration promoter, or a cranial neural progenitor cell homing promoter. The term "cranial neural progenitor cell homing promoter" means an agent that promotes migration of neural progenitor cells to a brain disorder site. A publicly known method may be employed to confirm that homing of cranial neural progenitor cells has been effectively achieved. For example, a patient or livestock animal promoter having nerve injury and having been administered a cranial neural progenitor cell homing promoter may be compared with a patient or livestock animal having nerve injury and not having been administered a cranial neural progenitor cell homing promoter, and it may be judged that efficient homing of cranial neural progenitor cells has been achieved if the patient or livestock animal that has been administered the cranial neural progenitor cell homing promoter is observed to have more migration of cranial neural progenitor cells to the brain disorder site from the hippocampal granule subcellular zone or the subventricular zone. Administration in combination with cells such as neural progenitor cells allows assessment to be made by accumulation of the administered cells in the brain disorder site as well. Homing of cranial neural progenitor cells can be evaluated using a cranial neural progenitor cell marker (such as DCX (doublecortin), SOX2 (SRY (sex determining region Y)-box 2) or PAX6 (paired box 6)), as described in the Examples below.

The fetal appendage-derived tissue cell culture supernatant of the disclosure may be a cranial nerve regeneration promoter, or a cranial neural progenitor cell differentiation/maturation promoter. The term "cranial neural progenitor cell differentiation/maturation promoter" means an agent that promotes axon elongation of neural progenitor cells in the brain and their differentiation and maturation into neurons.

A publicly known method may be employed to confirm that differentiation and maturation of cranial neural progenitor cells has been effectively achieved. For example, elongation of axons of neural progenitor cells in the brain or derived from the brain by addition of the cranial neural progenitor cell differentiation/maturation promoter can be evaluated by observation or by measurement of the lengths of the extended axons, as in the Examples described below. Neural progenitor cells harvested from a living body, or cells differentiated from stem cells such as iPS cells, or a cell line (such as A172, B65, C6, KS-1, N2A, PC12, SH-SY5Y, SKN-BE2, T98G, U251, U87 or YH-13) may be used.

The cranial nerve regeneration promoter of the disclosure includes cytokines and chemokines. Cytokines and chemokines include Interleukin(IL)-6, C-X-C Motif Chemokine Ligand (CXCL)1, CXCL7, CXCL8 (IL-8) and C-C Motif Chemokine Ligand (CCL)2 (MCP-1), which are important for cranial nerve regeneration.

The cranial nerve regeneration promoter of the disclosure may also include one or more of the following proteins:
cytokines such as the TNF Superfamily Member 14 (LIGHT), Interleukin(IL)-1α, IL-1β, IL-2, IL-3, IL-4, IL-5, IL-7, IL-10, IL-12p40/70, IL-13, IL-15, IL-16, Interferon (IFN)-y, Tumor Necrosis Factor (TNF)-α and TNF-β;
chemokines such as C-X-C Motif Chemokine Ligand (CXCL)1/2/3 (GROα/β/γ), CXCL6, CXCL9 (MIG), CXCL10 (IP-10), CXCL12 (SDF-1), CXCL13, C-C Motif Chemokine Ligand (CCL)1, CCL4, CCL5, CCL7, CCL8, CCL11, CCL13, CCL15, CCL17, CCL18, CCL20 (LARC), CCL22, CCL23, CCL24, CCL26 and C-X3-C Motif Chemokine Ligand (CX3CL)1 (FKN);
growth factor/related factors such as Angiogenin, Brain Derived Neurotrophic Factor (BDNF), Epidermal Growth Factor (EGF), Fibroblast Growth Factor (FGF)-4, FGF-6, FGF-7, FGF-9, Fms Related Receptor Tyrosine Kinase-3 Ligand (FLT-3L), Glial Cell Derived Neurotrophic Factor (GDNF), Granulocyte-Macrophage Colony-Stimulating Factor (GM-CSF), Granulocyte Colony-Stimulating Factor (G-CSF), Hepatocyte Growth Factor (HGF), Insulin Like Growth Factor (IGF)-1, Insulin Like Growth Factor Binding Protein (IGFBP)-2, IGFBP-1, IGFBP-4, IGFBP-3, Platelet Derived Growth Factor (PDGF)-BB, Placental Growth Factor (PLGF), Transforming Growth Factor (TGF)-β1, TGF-β2, TGF-β3, Thrombopoietin (TPO), Stem Cell Factor (SCF), Macrophage Colony Stimulating Factor (M-CSF), Neurotrophin (NT)-3, NT-4 and Vascular Endothelial Growth Factor (VEGF)-A; and
other mediators such as Leptin, Leukemia Inhibitory Factor (LIF), Macrophage Migration Inhibitory Factor (MIF), Osteopontin (OPN), Osteoprotegerin (OPG), Oncostatin M (OSM) and Tissue Inhibitor of Metalloproteinases (TIMP)-1 and TIMP-2.

The medium used to prepare the cranial nerve regeneration promoter of the disclosure may include deep sea water. The term "deep sea water" generally refers to seawater at a location greater than a depth of 200 meters, which around the region of Greenland is the origin of vertically sinking sea currents on a global scale known as "plumes" which are produced by difference in salt concentration, and consists of seawater that has been moving over the Earth for many centuries without ever contacting the atmosphere. Deep sea water is virtually shielded from sunlight rays and is in a low temperature high-pressure state, free of bacteria and rich in inorganic nutrient salts (minerals). By including deep sea water it is possible to adjust the amount of secretory proteins in culture supernatant and to prepare a more highly active cranial nerve disorder therapeutic agent than when deep sea water is not included. The concentration (v/v) of deep sea water in the medium may be, for example, 1%, 2%, 3%, 4%, 5%, 6%, 7%, 8%, 9%, 10%, 15%, 20%, 25%, 30%, 40%, 50%, 60%, 70% or 80% or greater, or it may be 90%, 80%, 70%, 60%, 50%, 45%, 40%, 35%, 30%, 25%, 20%, 15% or 10% or lower, such as in the range of 1 to 85%, 5 to 85%, 10% to 85%, 15% to 85%, 20% to 85%, 25% to 85%, 30% to 85%, 40% to 85%, 50% to 85%, 60% to 85%, 70% to 85%, 80% to 85%, 1 to 75%, 5 to 75%, 10% to 75%, 15% to 75%, 20% to 75%, 25% to 75%, 30% to 75%, 40% to 75%, 50% to 75%, 60% to 75%, 70% to 75%, 1 to 45%, 5 to 45%, 10% to 45%, 15% to 45%, 20% to 45%, 25% to 45%, 30% to 45%, 40% to 45%, 1 to 10% or 5 to 10%, or it may be 73% or 81%.

The culturing time for obtaining the culture supernatant of tissue cells derived from fetal appendage as a cranial nerve regeneration promoter may be, for example, 5 hours to 7 days, 1 day to 6 days, 1 day to 5 days, 1 day to 4 days, 1 day to 3 days or 1 day to 2 days, or it may be 0.5 day, 1 day, 2 days, 3 days, 4 days, 5 days, 6 days or 7 days. The culturing temperature may be 36°C to 38°C, such as 37°C, and the CO₂ concentration may be 4 to 6%, such as 5%, for example. The culturing may be three-dimensional culturing under non-adhesive conditions, for example, or suspension culturing (such as dispersion culturing or aggregated suspension culturing).

The density of tissue cells derived from fetal appendage at the start of culturing, to obtain a culture supernatant of tissue cells derived from fetal appendage as a cranial nerve regeneration promoter, may be 1 × 10⁴ to 1 × 10⁷/mL, 5 × 10⁴ to 5 × 10⁶/mL, 1 × 10⁵ to 5 × 10⁶/mL, 2 × 10⁵ to 5 × 10⁶/mL, 5 × 10⁵ to 5 × 10⁶/mL or 1 × 10⁶ to 5 × 10⁶/mL, such as 2 × 10⁵/mL, 3 × 10⁵/mL, 4 × 10⁵/mL, 5 × 10⁵/mL, 6 × 10⁵/mL, 7 × 10⁵/mL, 8 × 10⁵/mL, 9 × 10⁵/mL, 1 × 10⁶/mL, 2 × 10⁶/mL, 3 × 10⁶/mL, 4 × 10⁶/mL, 5 × 10⁶/mL, 6 × 10⁶/mL, 7 × 10⁶/mL, 8 × 10⁶/mL, 7 × 10⁶/mL, 8 × 10⁶/mL, 9 × 10⁶/mL or 1 × 10⁷/mL.

Since the cranial nerve regeneration promoter of the disclosure promotes nerve regeneration it can be used for a wide range of types of cranial nerve disorder. Non-hereditary cranial nerve disorder is an example of cranial nerve disorder. Without being limitative, the cranial nerve disorder therapeutic agent of the disclosure can be applied for dyskinesia (levodopa-induced dyskinesia, chronic or tardive dyskinesia, or orofacial dyskinesia), restless leg syndrome (drug-induced or idiopathic), drug-induced dystonia, chorea (Huntington's disease, toxin-induced chorea, Sydenham chorea, chorea of pregnancy, Wilson's disease, drug-induced chorea or metabolic or endocrine chorea), facial spasm (such as mobile, phonetic, simple, complex or Tourette's syndrome), dystonia (such as acute, systemic, localized, segmental, sexual, neutral, psychogenic or acute dystonic reaction), Sodemytopic Parkinson's disease, stereotypic movement disorder (such as autism or heredity or childhood-related movement disorder), obsessive-compulsive disorder, narcolepsy (such as cataplexy), transmissible spongiform encephalopathy (such as Creutzfeldt-Jakob disease or kuru), dementia (such as Alzheimer's, Lewy body dementia, vascular dementia, Pick's disease or alcoholic dementia), neuroacanthocytosis, seizure or convulsion, athetosis (such as Huntington's disease, respiratory arrest, neonatal jaundice- or stroke-related), or cerebral palsy.

According to one embodiment, a brain disorder such as signs or symptoms of cerebral palsy to be treated by the cranial nerve regeneration promoter of the disclosure is motor function impairment, language or speech dysfunction, ambulation or mobile dysfunction (such as reduced walking speed), dysfunctional muscle strength, dysfunctional muscle tone, abnormal reflex, abnormal coordination, spasticity, involuntary movement, abnormal ambulation, abnormal balance, reduced muscle mass, impaired skeletal development or impaired muscle development. According to one embodiment, the brain disorder, such as signs or symptoms of cerebral palsy, to be treated by the cranial nerve regeneration promoter of the disclosure is hand strength impairment, manual dexterity impairment, walking speed impairment or gait disturbance. According to one embodiment, the brain disorder such as signs or symptoms of cerebral palsy to be treated by the cranial nerve regeneration promoter of the disclosure is sensory motor disturbance or sensory motor function impairment, including but not limited to ataxia, systemic control disorder, impaired coordination or balance, impaired body sensation, impaired proprioceptive sensation, impaired endurance, impaired hand function, impaired hand strength, loss or impairment of fine hand coordination, hyperreflexia, impaired grip strength, reduced muscle strength, impaired muscle tone, impaired range of motion, spasticity, impaired or weakened strength, tremor, impaired limb function, upper extremity dysfunction, lower extremity dysfunction, impaired lower extremity muscle strength, gait disturbance (such as reduced walking speed), impaired ability to stand, impaired speech (such as stammering), impaired jaw function, chewing impairment, temporomandibular joint impairment, impaired dexterity, reflection, or impairment of any other sensory motor functions mentioned herein or known in the technical field.

The disclosure provides a method for producing a cranial nerve regeneration promoter which includes:
(a) a step of culturing tissue cells derived from fetal appendage, and (b) a step of recovering the culture supernatant of the tissue cells derived from fetal appendage. The production method may further include a step of carrying out one or more processes selected from among centrifugation, concentration, solvent exchange, dialysis, freezing, drying, freeze-drying, dilution and desalting of the collected culture supernatant. Including these steps will further facilitate handling, storage and transport of the cranial nerve regeneration promoter. The production method may also include a step of adding additional components to the collected culture supernatant. Addition of other components can alter the physical properties and improve the properties of the composition for promoting cranial nerve regeneration. The production method may also further include a step of preparing tissue cells derived from fetal appendage, from the tissue derived from fetal appendage. The conditions described for the cranial nerve disorder therapeutic agent of the disclosure all apply for each of these steps and additional components. When the method includes both a step of carrying out one or more processes selected from among centrifugation, concentration, solvent exchange, dialysis, freezing, drying, freeze-drying, dilution and desalting of the collected culture supernatant, and a step of adding additional components to the collected culture supernatant, the steps may be carried out in any order, or when possible they may be carried out simultaneously in parallel.

The cranial nerve regeneration promoter of the disclosure may also include other components for a pharmaceutical composition, for the purpose of supporting the expected curative effect in light of the condition of the subject to which it is to be applied. Examples of additional components include the following.

### (i) Bioabsorbable materials

Hyaluronic acid, collagen and fibrinogen (such as BOLHEAL^{™}) may be used as organic bioabsorbable materials.

### (ii) Gelling materials

Gelling materials are preferably highly biocompatible materials, such as hyaluronic acid, collagen or fibrin paste. Various types of hyaluronic acid or collagen may be selected for use, but they are preferably selected as suited for the purpose of use (the target tissue) of the cranial nerve regeneration promoter of the disclosure. The collagen used is preferably soluble (acid-soluble collagen, alkali-soluble collagen or enzyme-soluble collagen).

### (iii) Other components

Other pharmaceutically acceptable components (for example, carriers, excipients, disintegrators, buffering agents, emulsifying agents, suspending agents, soothing agents, stabilizers, preservatives, antiseptic agents, physiological saline and the like) may also be added. Excipients include lactose, starch, sorbitol, D-mannitol and saccharose. Disintegrators that may be used include starches, carboxymethyl cellulose and calcium carbonate. Buffering agents that may be used include phosphates, citrates and acetates. Emulsifying agents that may be used include gum arabic, sodium alginate and tragacanth. Suspending agents that may be used include glycerin monostearate, aluminum monostearate, methyl cellulose, carboxymethyl cellulose, hydroxymethyl cellulose and sodium lauryl sulfate. Soothing agents that may be used include benzyl alcohol, chlorobutanol and sorbitol. Stabilizers that may be used include propylene glycol and ascorbic acid. Preservatives that may be used include phenol, benzalkonium chloride, benzyl alcohol, chlorobutanol and methylparaben. Antiseptic agents that may be used include benzalkonium chloride, paraoxybenzoic acid and chlorobutanol. Antibiotics, pH adjustors, growth factors (such as epidermal growth factor (EGF), nerve growth factor (NGF) and brain-derived neurotrophic factor (BDNF)) may also be added.

The final form of the pharmaceutical composition of the cranial nerve regeneration promoter of the disclosure is not particularly restricted. Examples of dosage forms include liquid forms (liquids and gels) and solid forms (including lyophilized preparations such as powders, fine grains and granules). The pharmaceutical composition of the disclosure may also be in a form suited for inhalation, in which case it may be in a liquid form that is dispersible as a mist by a nebulizer or diffuser. The site of cranial nerve disorder is the brain, and in light of the presence of the blood brain barrier, the composition for promoting cranial nerve regeneration according to the disclosure is preferably in a form that can be administered intranasally, intracerebroventricularly or intrathecally. For example, it may be administered intranasally in the form of a spray or powder. From the viewpoint of prior preparation and storage, the culture supernatant of tissue cells derived from fetal appendage is more advantageous than using tissue cells derived from fetal appendage or stem cells derived from fetal appendage, for example, and may therefore be considered especially suitable for treatment of acute or subacute cranial nerve disorder. The culture supernatant of tissue cells derived from fetal appendage is also highly useful from the viewpoint of overcoming immunological rejection as well, since it does not contain cell components.

### EXAMPLES

The present invention will now be explained in greater detail by examples. However, the invention is in no way limited by the Examples.

### Example 1: Preparation of tissue cells derived from fetal appendage, and culture supernatant

### 1) Preparation of Walton colloid-derived mesenchymal cells

Human umbilical cord provided by Kochi University Medical School Hospital, Department of Obstetrics and Gynecology, with informed consent, was immersed for several seconds in 70% ethanol for sterilization, and phosphate buffer (D-PBS) was used for washing removal of the surrounding adhering blood. The umbilical cord was then incised, the navel vein and navel artery were removed, and the Walton colloid was cut out from the surrounding umbilical cord membrane. After chopping the Walton colloid, 2 g of tissue slices were aligned on a 10 cm plastic dish. Cell culture mesh Cellamigo^{™} (Tsubakimoto Chain) was placed over the cell strips and MEMα (containing 10% FBS, 100 µ/mL penicillin and 100 µg/mL streptomycin) was added prior to culturing at 37°C, 5% CO₂. The adhering cells that migrated and proliferated from the tissue slices were subcultured 2 to 5 times and used as Walton colloid-derived mesenchymal cells.

### 2) Preparation of amnionic mesenchymal cells

Human egg membrane provided by Kochi University Medical School Hospital, Department of Obstetrics and Gynecology, with informed consent, was immersed for several seconds in 70% ethanol for sterilization, and the membrane was detached by a common method. After collecting the first layer as amnion from the egg membrane fetus side, the membrane structure was confirmed by hematoxylin-eosin staining. This was immersed in [0.05% Trypsin/0.2 mM EDTA] solution and reacted at 37°C for 1.5 hours, and the amnion epithelial tissue was dispersed. The remaining amniotic connective tissue was immersed in [2.4 mg/L collagenase, 0.01 mg/mL DNase] enzyme solution and reacted at 37°C for 1.5 hours, and the amniotic connective tissue was dispersed. The enzyme solution was removed by twice repeating a procedure of passing the amniotic connective tissue dispersion through a 70 µm cell strainer, centrifugation and supernatant removal of the strained solution and resuspension in D-PBS, and MEMα (containing 10% FBS, 100 µ/mL penicillin and 100 µg/mL streptomycin) was added to the obtained cells prior to culturing at 37°C, 5% CO₂. The proliferated cells were subcultured 2 to 5 times for use as amnionic mesenchymal cells.

### 3) Chorionic mesenchymal cells

Human egg membrane was immersed for several seconds in 70% ethanol for sterilization, and the membrane was detached by a common method. After collecting the second layer as chorionic connective tissue from the egg membrane fetus side, the membrane structure was confirmed by hematoxylin-eosin staining. The remaining chorionic connective tissue was immersed in [2.4 mg/L collagenase, 0.01 mg/mL DNase] enzyme solution and reacted at 37°C for 1.5 hours, and the chorionic connective tissue was dispersed. A procedure of passing the chorionic connective tissue dispersion through a 100 µm cell strainer, centrifugation and supernatant removal of the strained solution and resuspension in D-PBS was repeated twice. After further immersion in a [0.05% trypsin/0.2 mM EDTA] solution and reaction at 37°C for 5 minutes, MEMα (containing 10% FBS, 100 µ/mL penicillin and 100 µg/mL streptomycin) was added to halt the reaction. The enzyme solution was removed by twice repeating a procedure of passing the trypsin dispersion through a 70 µm cell strainer, centrifugation and supernatant removal of the strained solution and resuspension in D-PBS, and MEMα (containing 10% FBS, 100 µ/mL penicillin and 100 µg/mL streptomycin) was added to the obtained cells prior to culturing at 37°C, 5% CO₂. The proliferated cells were subcultured 2 to 5 times for use as chorionic mesenchymal cells.

### 4) Culturing of tissue cells derived from fetal appendage

Human Walton colloid-derived mesenchymal cells, human amnionic mesenchymal cells or human chorionic mesenchymal cells were suspended in DMEM medium or RPMI 1640 medium at 2 × 10⁵/mL, and cultured for 24 hours under conditions of 37°C, 5% CO₂, either with or without of addition of deep sea water. The deep sea water used was "Amami Water 1000" (Ako Kasei Co., Ltd.) taken offshore of Muroto City, Kochi prefecture. A NucleoCounter NC-100 (ChemoMetec Co.) was used to measure the cell viability, and while the results showed time-dependent reduction in survival rate, no difference was seen between addition and non-addition of the deep sea water. The medium was recovered and centrifuged at 300 G for 10 minutes to prepare a culture supernatant.

### Example 2: Nerve regeneration by co-culturing of tissue cells derived from fetal appendage and neural progenitor cells

### 1) Preparation of neural progenitor cells

Neonatal NOD/SCID mice (Charles River, Japan: NOD.CB17-Prkdc ^{scid}/J) were euthanized 1 week after birth, and brain tissue was harvested. Surrounding tissue in the subventricular zone was cut out and dispersed using neuronal dispersion (Sumitomo Dainippon Pharma) and the obtained cells were cultured in NeuroCult growth medium (Stemcell Technologies) with addition of 20 ng/mL epidermal growth factor (EGF), 10 ng/mL fibroblast growth factor (FGF) and 2 µL /mL heparin (Stemcell Technologies) at 37°C, 5% CO₂, forming a neural progenitor cell mass which was provided for the subsequent test.

### 2) Co-culturing

The human Walton colloid-derived mesenchymal cells prepared in Example 1 and the mouse neural progenitor cells prepared in Example 2 were each suspended in NeuroCult Basal complete medium (Stemcell Technologies), and a µ-Slide Chemotaxis (Ibidi Co.) was used for co-culturing under conditions of 37°C, 5% CO₂, without contact between the cells. Proliferation and differentiation/maturation of the neural progenitor cells were evaluated on the 3rd and 7th days of culturing by microscope observation. As shown in Fig. 1, the Walton colloid-derived mesenchymal cells and co-cultured neural progenitor cells promoted maintenance of proliferation or survival of neural progenitor cells, with observation of greater movement (migration) of neural progenitor cells, compared to culturing alone, while greater differentiation and maturation to axon-extended neurons was also observed.

These results suggested that Walton colloid-derived mesenchymal cells in the prepared state (i.e. with all of the cell types including stem cells present without being isolated) secrete factors that promote proliferation, survival, movement (migration), differentiation and maturation of neural progenitor cells.

### Example 3: Nerve regeneration (in vitro) by fetal appendage-derived tissue cell culture supernatant

### 1) Effect on neural progenitor cells

The neural progenitor cells prepared in Example 2 were suspended in NeuroCult Basal complete medium (Stemcell Technologies) and seeded in a µ-Slide Chemotaxis (Ibidi Co.), and culture supernatant of the human Walton colloid-derived mesenchymal cells obtained in Example 1 was added at 50% (v/v) prior to culturing under conditions of 37°C, 5% CO₂. As a control, the same medium used for preparation of the Walton colloid-derived mesenchymal cell culture supernatant was added in the same amount. Proliferation and differentiation/maturation of the neural progenitor cells were evaluated on the 3rd and 7th days of culturing by microscope observation. As shown in Fig. 2, compared to the control, the neural progenitor cells to which culture supernatant of Walton colloid-derived mesenchymal cells was added showed greater extension of axons on the 3rd day of culturing and greater proliferation or survival maintenance of the cells on the 3rd and 7th days of culturing, as well as greater movement (migration) of the neural progenitor cells.

These results suggested that Walton colloid-derived mesenchymal cells in the prepared state (i.e. with all of the cell types including stem cells present without being isolated) secrete factors that promote proliferation, survival, movement (migration), differentiation and maturation of neural progenitor cells, and that Walton colloid-derived mesenchymal cell culture supernatant promotes nerve regeneration.

### 2) Effect on neural progenitor cells (2)

The human neuroblastoma cell line SH-SY5Y (ATCC No.: CRL-2226), as a neural progenitor cell line, in DMEM culture solution at a concentration of 2 × 10⁵ cells/mL, was seeded at 100 µL into each well of a 96-well plastic plate. The culture supernatants prepared in Example 1 were added at 100 µL and cultured for 5 days under conditions of 37°C, 5% CO₂. As a control, culturing was carried out with addition of the same amount of the medium used for preparation of each culture supernatant. The cultured cells were photographed with a phase contrast microscope and the neurite lengths were measured using analysis software ImageJ (NIH).

While the average neurite length in the control group was 29 µm, when culture supernatant of human Walton colloid-derived mesenchymal cells, human amnionic mesenchymal cells or human chorionic mesenchymal cells was added the average neurite length was 62 µm, 43 µm and 41 µm (n = 3 wells), respectively, suggesting that fetal appendage-derived tissue cell culture supernatants promote axon elongation of neural progenitor cells and promote nerve differentiation.

These results suggested that tissue cells derived from fetal appendage in the prepared state (i.e. with all of the cell types including stem cells present without being isolated) secrete factors that promote survival maintenance, differentiation and maturation of neural progenitor cells, and that culture supernatant of tissue cells derived from fetal appendage promote nerve regeneration.

### 3) Factors in culture supernatants

The factors included in the culture supernatants of Walton colloid-derived mesenchymal cells, amnionic mesenchymal cells and chorionic mesenchymal cells prepared in Example 1 were examined by antibody array (Human Cytokine Array C5 by RayBiotech) (n = 3). As shown in Tables 1 to 3, cytokines such as IL-6, chemokines such as CCL2, CXCL1, CXCL5, CXCL7, CXCL8 and GROα/β/γ, growth factors such as BDNF and EGF, and Osteopontin (OPN) were present in the culture supernatants, suggesting that these factors promote nerve regeneration. When culture supernatants were prepared in medium with addition of deep sea water at 8, 41, 73 and 81%, the concentrations of some or all of these factors increased.

**[Table 1]**

| Factors in Walton colloid-derived mesenchymal cell culture supernatant | | | | | | | |
|---|---|---|---|---|---|---|---|
| Factor | Relative concentration | Factor | Relative concentration | Factor | Relative concentration | Factor | Relative concentration |
| Angiogenin | 136 | CXOL5 | 182 | IGFBP-1 | 72 | LIGHT | 59 |
| BDNF | 76 | CXCL6 | 144 | IGFBP-2 | 131 | M-CSF | 83 |
| CCL1 | 27 | CXCL7 | 96 | IGFBP-3 | 116 | MIF | 103 |
| CCL2 | 1297 | CXCL8 | 1396 | IGFBP-4 | 114 | NT3 | 244 |
| CCL4 | 107 | OXCL9 | 86 | IL-1a | 40 | NT-4 | 126 |
| CCL5 | 64 | CXCL10 | 157 | IL-1b | 47 | OPG | 377 |
| CCL7 | 57 | CXCL12 | 36 | IL-2 | 37 | OPN | 405 |
| CCL8 | 38 | CXCL13 | 53 | IL-3 | 51 | OSM | 78 |
| CCL11 | 64 | EGF | 62 | IL-4 | 52 | PDGF-BB | 43 |
| COL13 | 76 | FGF-4 | 55 | IL-5 | 45 | PLGF | 63 |
| CCL15 | 29 | FGF-6 | 85 | IL-6 | 1462 | SCF | 40 |
| CCL17 | 77 | FGF-7 | 38 | IL-7 | 68 | TGF-b1 | 31 |
| COL18 | 81 | FGF-9 | 79 | IL-10 | 74 | TGF-b2 | 278 |
| CCL20 | 70 | FLT-3L | 28 | IL-12 | 38 | TGF-b3 | 86 |
| CCL22 | 74 | G-CSF | 32 | IL-13 | 25 | TIMP-1 | 373 |
| CCL23 | 50 | GDNF | 56 | IL-15 | 59 | TIMP-2 | 1101 |
| CCL24 | 83 | GM-CSF | 67 | IL-16 | 54 | TNF-a | 46 |
| CCL26 | 81 | GRO a/b/g | 349 | INF-g | 47 | TNF-b | 72 |
| CX3GL1 | 38 | HGF | 101 | Leptin | 36 | TPO | 37 |
| CXCL1 | 577 | IGF-1 | 80 | LIF | 148 | VEGF-A | 52 |

**[Table 2]**

| Factors in amnionic mesenchymal cell culture supernatant | | | | | | | |
|---|---|---|---|---|---|---|---|
| Factor | Relative concentration | Factor | Relative concentration | Factor | Relative concentration | Factor | Relative concentration |
| Angiogenin | 137 | CXCL5 | 166 | IG FBP-1 | 20 | LIGHT | 20 |
| BDNF | 48 | CXCL6 | 45 | IGFBP-2 | 83 | M-CSF | 50 |
| CCL1 | 16 | CXCL7 | 38 | IGFBP-3 | 68 | MIF | 87 |
| CCL2 | 1137 | CXCL8 | 888 | IGFBP-4 | 42 | NT-3 | 147 |
| CCL4 | 86 | CXCL9 | 42 | IL-1a | is | NT-4 | 49 |
| CCL5 | 20 | CXCL10 | 130 | IL-1b | 20 | OPG | 102 |
| CCL7 | 43 | CXGL12 | 7 | IL-2 | 22 | OPN | 196 |
| CCL8 | 18 | CXOL13 | 14 | IL-3 | 30 | OSM | 34 |
| CCL11 | 21 | EGF | 25 | IL-4 | 22 | PDGF-BB | 25 |
| CCL1 3 | 19 | FGF-4 | 16 | IL-5 | 14 | PLGF | 28 |
| CCL15 | 6 | FGF-6 | 40 | IL-6 | 847 | SCF | 12 |
| CCL17 | 43 | FGF-7 | 15 | IL-7 | 28 | TGF-b1 | 19 |
| CCL18 | 40 | FGF-9 | 54 | IL-10 | 53 | TGF-b2 | 223 |
| CCL20 | 25 | FLT-3L | 14 | IL-12 | 14 | TGF-b3 | 34 |
| CCL22 | 39 | G-CSF | 16 | IL-13 | 4 | TIMP-1 | 319 |
| CCL23 | 18 | GDNF | 18 | IL-15 | 28 | TIMP-2 | 1249 |
| CCL24 | 31 | GM-CSF | 34 | IL-16 | 20 | TNF-a | 31 |
| CCL26 | 28 | GRO a/b/g | 85 | INF-g | 30 | TNF-B | 47 |
| CX3CL1 | 15 | HGF | 19 | Leptin | 16 | TPO | 15 |
| CXCL1 | 423 | IGF-1 | 28 | LIF | 96 | VEGF-A | 27 |

**[Table 3]**

| Factors in chorionic mesenchymal cell culture supernatant | | | | | | | |
|---|---|---|---|---|---|---|---|
| Factor | Relative concentration | Factor | Relative concentration | Factor | Relative concentration | Factor | Relative concentration |
| Angiogenin | 100 | CXCL5 | 44 | IGFBP-1 | 43 | LIGHT | 44 |
| BDNF | 106 | CXCL6 | 33 | IGFBP-2 | 73 | M-CSF | 82 |
| CCL1 | 32 | CXCL7 | 55 | IGFBP-3 | 68 | MIF | 77 |
| CCL2 | 1264 | CXCL8 | 1128 | IGFBP-4 | 44 | NT3 | 186 |
| CCL4 | 76 | CXCL9 | 48 | IL-1a | 29 | NT-4 | 27 |
| CCL5 | 58 | CXCL10 | 224 | IL-1b | 39 | OPG | 234 |
| CCL7 | 41 | CXCL12 | 45 | IL-2 | 49 | OPN | 107 |
| CCL8 | 41 | CXCL13 | 38 | IL-3 | 55 | OS M | 63 |
| CCL11 | 48 | EGF | 43 | IL-4 | 43 | PDGF-BB | 54 |
| CCLL3 | 46 | FGF-4 | 14 | IL-5 | 26 | PLGF | 63 |
| CCL15 | 17 | FGF-6 | 40 | IL-6 | 2104 | SCF | 53 |
| CCL17 | 99 | FGF-7 | 18 | IL-7 | 50 | TGF-b1 | 46 |
| CCL18 | 77 | FGF-9 | 83 | IL-10 | 51 | TGF-b2 | 288 |
| COL20 | 47 | FLT-3L | 20 | IL-12 | 32 | TGF-b3 | 61 |
| CCL22 | 56 | G-CSF | 28 | IL-13 | 17 | TIMP-1 | 386 |
| CCL23 | 46 | GDNF | 41 | IL-15 | 75 | TIMP-2 | 1125 |
| CCL24 | 47 | GM-CSF | 58 | IL-16 | 29 | TNF-a | 56 |
| CCL26 | 35 | GRO a/b/g | 54 | INF-g | 70 | TNF-b | 83 |
| CX3CL1 | 25 | HGF | 405 | Leptin | 29 | TPO | 19 |
| GXCL1 | 212 | IGF-1 | 42 | LIF | 139 | VEGF-A | 49 |

### Example 4: Nerve regeneration (in vivo) by fetal appendage-derived tissue cell culture supernatants

### 1) Creation of mouse hypoxic reperfusion pediatric cerebral palsy model

ICR mice (Crl: CD1, Charles River, Japan) were used to create hypoxic reperfusion pediatric cerebral palsy model mice based on the method of Rice-Vannucci et al. (Wang et al., J Matern Fetal Neonatal Med. 2015; 28(7):842-7). Mice (female and male) at 1 week (7-9 days) after birth were anesthetized with 2% isoflurane, and the right carotid arteries were infarcted with a cerebral aneurysm clip (Mizuho Co.). Each individual was left for 120 minutes under 8% oxygen concentration, after which the cerebral artery clip was released and reperfusion of blood flow was initiated. Mice that survived for 3 weeks with stabilized brain disorder symptoms were provided for the following experiment.

### 2) Treatment with Walton colloid-derived mesenchymal cell culture supernatant (1)

Hypoxic pediatric paralysis model mice were intranasally administered 10 µL of the Walton colloid-derived mesenchymal cell culture supernatant prepared in Example 1, every other day for a period of 2 weeks. After treatment, the mice were used in a Y-maze test (YM-3002, by O'Hara & Co., Ltd.), measuring the total entry count as an indicator of spontaneous behavior and the alternation rate as an indicator of short-term working memory (n = 4), during free activity for 8 minutes. As shown in Fig. 3, spontaneous behavior and short-term working memory were improved after 2 weeks of treatment in the culture supernatant-administered group.

### 3) Treatment with Walton colloid-derived mesenchymal cell culture supernatant (2)

Hypoxic pediatric paralysis model mice were intranasally administered 10 µL of the Walton colloid-derived mesenchymal cell culture supernatant prepared in Example 1, every other day for a period of 2 weeks. The treated mice were used in a Rotarod test (MK-610A by Muromachi Kikai Co., Ltd.), measuring the time until falling from a rotor accelerated to 80 rpm in 300 seconds, and a tendency toward recovery from the baseline of one week after treatment was exhibited in the culture supernatant-administered group (ambulation times of 67, 34 and 50% before treatment, 1 week after treatment and 2 weeks after treatment, respectively, with respect to the ambulation time of healthy mice (n = 2)).

The therapeutic effect of Walton colloid-derived mesenchymal cell culture supernatant over a longer period was confirmed next. Hypoxic pediatric paralysis model mice were randomly divided into two groups, and those in the treatment group were intranasally administered 10 µL of the Walton colloid-derived mesenchymal cell culture supernatant prepared in Example 1, every other day for a period of 2 weeks (n = 5). The mouse were used in a Rotarod test (MK-610A by Muromachi Kikai Co., Ltd.) before and after treatment, measuring the time until falling from a rotor accelerated to 40 rpm in 300 seconds. Compared to the control group (non-treatment group: n = 2), the group treated with Walton colloid-derived mesenchymal cell culture supernatant for two weeks showed significant recovery (2W: p < 0.05, Tukey statistical test), which was equivalent to the healthy mice (n = 7). The therapeutic effect also continued for 2 weeks after drug withdrawal (4W) (Fig. 4).

It was thus demonstrated that tissue cells derived from fetal appendage in the prepared state (i.e. with all of the cell types including stem cells present without being isolated) secrete factors that promote proliferation, survival, homing, differentiation and maturation of neural progenitor cells, and that culture supernatant of tissue cells derived from fetal appendage can be used to regenerate nerves and to treat cranial nerve disorders such as cerebral palsy.

## Claims

1. A cranial nerve disorder therapeutic agent comprising a culture supernatant of tissue cells derived from fetal appendage as an active ingredient.

2. The cranial nerve disorder therapeutic agent according to claim 1, wherein the tissue cells derived from fetal appendage are umbilical cord cells, placental cells, egg membrane cells, chorionic cells, amniotic cells or a combination thereof.

3. The cranial nerve disorder therapeutic agent according to claim 1 or 2, wherein the tissue cells derived from fetal appendage are Walton colloid-derived mesenchymal cells, amnionic mesenchymal cells or chorionic mesenchymal cells, or a combination thereof.

4. The cranial nerve disorder therapeutic agent according to any one of claims 1 to 3, wherein the culture supernatant comprises cytokines and chemokines.

5. The cranial nerve disorder therapeutic agent according to any one of claims 1 to 4, comprising IL-6, CXCL1, CXCL7, CXCL8 and CCL2.

6. The cranial nerve disorder therapeutic agent according to any one of claims 1 to 5, wherein the culture supernatant is obtained by culturing tissue cells derived from fetal appendage for 1 to 3 days.

7. The cranial nerve disorder therapeutic agent according to any one of claims 1 to 6, wherein the density of tissue cells derived from fetal appendage is 5 × 10⁴ to 5 × 10⁶/mL at the start of culturing.

8. The cranial nerve disorder therapeutic agent according to any one of claims 1 to 7, wherein the cranial nerve disorder is non-hereditary cranial nerve disorder.

9. The cranial nerve disorder therapeutic agent according to any one of claims 1 to 8, wherein the cranial nerve disorder is dyskinesia (levodopa-induced dyskinesia, chronic or tardive dyskinesia, or orofacial dyskinesia), restless leg syndrome (drug-induced or idiopathic), drug-induced dystonia, chorea (Huntington's disease, toxin-induced chorea, Sydenham chorea, chorea of pregnancy, Wilson's disease, drug-induced chorea, or metabolic or endocrine chorea), facial spasm (such as mobile, phonetic, simple, complex or Tourette's syndrome), dystonia (such as acute, systemic, localized, segmental, sexual, neutral, psychogenic or acute dystonic reaction), Sodemytopic Parkinson's disease, stereotypic movement disorder (such as autism or heredity or childhood-related movement disorder), obsessive-compulsive disorder, narcolepsy (such as cataplexy), transmissible spongiform encephalopathy (such as Creutzfeldt-Jakob disease or kuru), dementia (such as Alzheimer's, Lewy body dementia, vascular dementia, Pick's disease or alcoholic dementia), neuroacanthocytosis, seizure or convulsion, athetosis (such as Huntington's disease, respiratory arrest, neonatal jaundice- or stroke-related) or cerebral palsy.

10. A method for producing a cranial nerve disorder therapeutic agent according to any one of claims 1 to 9, the method including (a) a step of culturing tissue cells derived from fetal appendage, and (b) a step of recovering the culture supernatant of the tissue cells derived from fetal appendage.

11. A pharmaceutical composition comprising a cranial nerve disorder therapeutic agent according to any one of claims 1 to 9.

12. The pharmaceutical composition according to claim 11, which is a lyophilized preparation.

13. A cranial nerve regeneration promoter comprising a culture supernatant of tissue cells derived from fetal appendage as an active ingredient.

14. The cranial nerve regeneration promoter according to claim 13, wherein the tissue cells derived from fetal appendage are umbilical cord cells, placental cells, egg membrane cells, chorionic cells, amniotic cells or a combination thereof.

15. The cranial nerve regeneration promoter according to claim 13 or 14, wherein the tissue cells derived from fetal appendage are Walton colloid-derived mesenchymal cells, amnionic mesenchymal cells or chorionic mesenchymal cells, or a combination thereof.

16. The cranial nerve regeneration promoter according to any one of claims 13 to 15, wherein the cranial nerve regeneration promoter is a cranial neural progenitor cell proliferating/survival promoter, a cranial neural progenitor cell homing promoter, a cranial neural progenitor cell differentiation/maturation promoter, or a combination thereof.

17. The cranial nerve regeneration promoter according to any one of claims 13 to 16, wherein the culture supernatant comprises cytokines and chemokines.

18. The cranial nerve regeneration promoter according to any one of claims 13 to 17, comprising IL-6, CXCL1, CXCL7, CXCL8 and CCL2.

19. The cranial nerve regeneration promoter according to any one of claims 13 to 18, wherein the culture supernatant is obtained by culturing tissue cells derived from fetal appendage for 1 to 3 days.

20. The cranial nerve regeneration promoter according to any one of claims 13 to 19, wherein the density of postpartum tissue is 5 × 10⁴ to 5 × 10⁶/mL at the start of culturing.

21. The cranial nerve regeneration promoter according to any one of claims 13 to 20, wherein the cranial nerve regeneration occurs with non-hereditary cranial nerve disorder.

22. The cranial nerve regeneration promoter according to any one of claims 13 to 21, wherein the cranial nerve regeneration occurs with dyskinesia (levodopa-induced dyskinesia, chronic or tardive dyskinesia, or orofacial dyskinesia), restless leg syndrome (drug-induced or idiopathic), drug-induced dystonia, chorea (Huntington's disease, toxin-induced chorea, Sydenham chorea, chorea of pregnancy, Wilson's disease, drug-induced chorea or metabolic or endocrine chorea), facial spasm (such as mobile, phonetic, simple, complex or Tourette's syndrome), dystonia (such as acute, systemic, localized, segmental, sexual, neutral, psychogenic or acute dystonic reaction), Sodemytopic Parkinson's disease, stereotypic movement disorder (such as autism or heredity or childhood-related movement disorder), obsessive-compulsive disorder, narcolepsy (such as cataplexy), transmissible spongiform encephalopathy (such as Creutzfeldt-Jakob disease or kuru), dementia (such as Alzheimer's, Lewy body dementia, vascular dementia, Pick's disease or alcoholic dementia), neuroacanthocytosis, seizure or convulsion, athetosis (such as Huntington's disease, respiratory arrest, neonatal jaundice- or stroke-related) or cerebral palsy.

23. A method for producing a cranial nerve regeneration promoter according to any one of claims 13 to 22, the method including (a) a step of culturing tissue cells derived from fetal appendage, and (b) a step of recovering the culture supernatant of the tissue cells derived from fetal appendage.

24. A pharmaceutical composition comprising a cranial nerve regeneration promoter according to any one of claims 13 to 22.

25. The pharmaceutical composition according to claim 24, which is a lyophilized preparation.
